# EUROPÄISCHE PATENTSCHRIFT

(11) **EP 2 196 798 B1**
(45) Veröffentlichungstag und Bekanntmachung des Hinweises auf die Patenterteilung: **06.04.2011**
(21) Anmeldenummer: 08171059.2
(22) Anmeldetag: 09.12.2008
(51) Int. Cl.: G01N 27/447, G01N 33/18

(54) **Wasser-Prozessanalysegerät und Verfahren zur kontinuierlichen automatischen Bestimmung eines ionischen Wasser-Analyts in Wasser**
Water process analysis device and method for continuously automatically detecting an ionic water analyte in water
Appareil d'analyse de processus à l'eau et procédé de détermination automatique et continue d'un analyte d'eau ionique dans l'eau

(43) Veröffentlichungstag der Anmeldung: 16.06.2010
(73) Patentinhaber: Hach Lange GmbH, 14163 Berlin (DE)
(72) Erfinder: Farjam, Aria, 40223 Düsseldorf (DE); Lenhard, Markus, 41751 Viersen (DE); Uthemann, Rolf, 51373 Leverkusen (DE); Hahn, Markus Dr., 47906 Kempen (DE)
(74) Vertreter: Patentanwälte ter Smitten

(56) Entgegenhaltungen:
- DE-A1- 19 537 059
- US-A1- 2004 256 231
- PETR KUBAN, BO KARLBERG: "On-Line Dialysis Coupled to a Capillary Electrophoresis System for Determination of Small Anions" ANALYTICAL CHEMISTRY, Bd. 69, Nr. 6, 15. März 1997 (1997-03-15), Seiten 1169-1173, XP002515709
- DANFOSS: "EVITA - In situ sensors for measuring ammonium, nitrate or orthophosphate" 1. August 1997 (1997-08-01), DANFOSS DATA SHEET, XX, XX, PAGE(S) 4PAGES , XP002989216 * Seite 2 - Seite 3 *

## Beschreibung

Die Erfindung bezieht sich auf ein Wasser-Prozessanalysegerät zur automatischen quasi-kontinuierlichen Bestimmung eines ionischen Wasser-Analyts in Wasser.

Unter Wasser ist vorliegend beispielsweise Trinkwasser, Abwasser, Prozesswasser, Kesselwasser oder Oberflächenwasser zu verstehen.

Unter einem Prozessanalysegerät ist vorliegend ein Gerät zu verstehen, das in der Lage ist, den gesamten Messzyklus automatisch durchzuführen. Das Prozessanalysegerät dient aber nicht notwendigerweise einer Prozesssteuerung oder -kontrolle. Das Prozessanalysegerät kann auch im Labor benutzt werden, wobei beispielsweise die Probe in einem Probenbehälter vorgelegt wird. Über einen Schlauch oder eine Pipette kann die Probe vom Analysegerät angesaugt werden.

In der Wasser-Prozessanalyse zur Bestimmung eines Analyts, beispielsweise Ammonium, Phosphat oder Nitrat, wird zur Probenvorbereitung eine Dialysemembran zur Erzeugung eines Wasserdialysats eingesetzt. Dem auf diese Weise generierten Wasserdialysat werden anschließend ein oder mehrere Reagenzien zugeführt, das bzw. die mit dem zu bestimmenden Wasser-Analyt farbverändernd reagiert bzw. reagieren. Anschließend wird das Wasserdialysat einem Fotometer zugeführt, in dem das betreffende Wasser-Analyt qualitativ und quantitativ fotometrisch bestimmt wird. Viele der Reagenzien sind gesundheitsschädlich, giftig oder ätzend. Für jeden Analyten werden unterschiedliche Reagenzien und Reaktionsbedingungen benötigt.

Anschließend muss das Wasserdialysat, das für weitere fotometrische Bestimmungen anderer Analyte ungeeignet ist, schon wegen der gegebenenfalls umweltschädigenden Eigenschaft der zugeführten Reagenzien in einen Abfalltank entsorgt werden, der regelmäßig entleert werden muss. Zur Bestimmung eines Analyts mit dieser Methode muss stets ein entsprechendes Reagenz bereitgestellt werden, was bei einem Wasser-Prozessanalysegerät einen entsprechenden Vorratstank und eine regelmäßige Neubefüllung erfordert.

In der Druckschrift PETR KUBAN, BO KARLBERG: "On-Line Dialysis Coupled to a Capillary Electrophoresis System for Determination of Small Anions" ANALYTICAL CHEMISTRY, Bd. 69, Nr. 6, 15. März 1997 (1997-03-15), Seiten 1169-1173 ist ein Analysegerät zur kontinuierlichen Bestimmung eines Wasser-Analyts im Wasser bekannt, das eine Dialysemembran, ein Kapillarelektrophorese-Element und eine Probentransporteinrichtung zum Transport des Wasserdialysats durch eine Probenleitung von der Dialysemembran zu dem Kapillarelektrophorese-Element aufweist. Dem Kapillarelektrophorese-Element ist ein Analyt-Detektor zugeordnet, der das in dem Kapillarelektrophorese-Element getrennte Analyt detektiert.

Aus US 2004/0256231 ist ein Kapillarelektrophorese-System bekannt, das nach Durchführung einer Messung mit einer Spülflüssigkeit aus einem Spolflüssigkeits-Vorratsbehälter gespült werden kann.

Aufgabe der Erfindung ist es, ein Prozessanalysegerät mit hoher Messfrequenz, also zeitlich kurzen Messzyklen, zu schaffen.

Diese Aufgabe wird erfindungsgemäß gelöst durch ein Wasser-Prozessanalysegerät mit den Merkmalen des Patentanspruches 1.

Das erfindungsgemäße Wasser-Prozessanalysegerät weist eine Dialysemembran mit einer Wasserseite und einer Dialysatseite auf, wobei auf der Dialysatseite ein Wasserdialysat generiert wird. Ferner ist ein Kapillarelektrophorese-Element zur Auftrennung der Analyte in dem Wasserdialysat vorgesehen. Das Wasserdialysat wird durch eine Probentransport-Einrichtung automatisch über eine Probenleitung von der Dialysatseite der Membran zu dem Kapillarelektrophorese-Element transportiert. Dem Kapillarelektrophorese-Element ist ein Analyt-Detektor zugeordnet, der das in dem Kapillarelektrophorese-Element getrennte Analyt detektiert.

Bei der Kapillarelektrophorese werden die Ionen in dem Wasserdialysat in einem dünnen Kapillarrohr unter Einfluss eines elektrischen Feldes voneinander getrennt, und können nach vollzogener Trennung durch einen geeigneten Analyt-Detektor qualitativ und quantitativ bestimmt werden. Für die elektrophoretische Trennung ist grundsätzlich kein Reagenz erforderlich, so dass ein Reagenz-Vorratstank entfallen kann und folglich, auch eine regelmäßige Reagenz-Neubefüllung entfällt. Da auf ein möglicherweise umweltschädigendes Reagenz verzichtet wird, entfällt unter Umständen auch die Notwendigkeit, das Wasserdialysat, das im Wesentlichen nur aus einer Trägerflüssigkeit bzw. einem Puffer besteht, nach der Elektrophorese in einem Abfalltank zu speichern, der wiederum regelmäßig entleert werden müsste.

Durch den Verzicht auf eine das Wasserdialysat verändernde Reagenz und durch die der Elektrophorese immanente Eigenschaft, alle Ionen in Abhängigkeit von ihrer Masse, ihrer Größe, ihrer Ladung etc. zu trennen, können mit einer einzigen Elektrophorese mehrere Ionen, das heißt mehrere Analyte einer einzigen Wasserdialysat-Probe, bestimmt werden. Problematisch ist bei der Kapillarelektrophorese die Gefahr, dass die Elektrophorese-Kapillare schon durch kleinste Partikel verstopft werden kann bzw. durch die Ablagerung von gelösten organischen Molekülen, z. B. Proteine, Huminsäuren, in ihrer Trennleistung verändert oder sogar verstopft werden kann. Durch die Kombination der Kapillarelektrophorese mit einer Dialysemembran zur Herstellung der Wasserdialysat-Probe ist ausgeschlossen, dass kleine Partikel oder größere organische Moleküle über das Wasserdialysat in das Kapillarrohr gelangen können und dieses zusetzen.

Gemäß einer bevorzugten Ausgestaltung ist ein Trägerflüssigkeits-Vorratstank zur Versorgung der ionenselektiven und/oder größenselektiven Dialysemembran, und optional des Kapillarelektrophorese-Elements, mit einer Trägerflüssigkeit vorgesehen. Im Allgemeinen wird für eine kontaktlose Leitfähigkeitsmessung in dem Detektor eine Lösung mit niedriger Leitfähigkeit benötigt, so dass für die Dialyse im einfachsten Fall destilliertes Wasser bzw. der Trennpuffer, der in der Kapillarelektrophorese eingesetzt wird, verwendet werden kann. Die Trägerflüssigkeit nimmt die Analyten aus dem Wasser auf der Wasserseite, die durch die Dialysemembran zur Dialysatseite gelangen, auf. Die Trägerflüssigkeit transportiert die Ionen zu dem Kapillarelektrophorese-Element. Die Trägerflüssigkeit ist idealerweise nicht umweltschädlich, so dass sie nicht in einen Abfalltank, sondern nach außen in das umgebende Wasser abgeleitet werden kann.

Vorzugsweise ist zusätzlich ein pH-Pufferlösungs-Vorratstank vorgesehen, dessen Pufferlösung zwischen der Dialysemembran und dem Kapillarelektrophorese-Element in die Probenleitung eingeleitet wird. Die Wanderungsbewegung von Ionen in dem Kapillarelektrophorese-Element ist unter anderem stark abhängig vom pH-Wert. Um reproduzierbare Ergebnisse bei der Kapillarelektrophorese sicherzustellen, muss der pH-Wert bei allen Messungen annähernd gleich sein. Durch Zuführung einer relativ kleinen Menge einer möglichst konzentrierten pH-Pufferlösung zu dem Wasserdialysat wird ein stets konstanter pH-Wert des auf diese Weise geringfügig verdünnten Wasserdialysats sichergestellt. Die pH-Pufferlösung wird in dem Analysegerät in dem pH-Pufferlösungs-Vorratstank bereitgestellt.

Gemäß einer bevorzugten Ausgestaltung ist ein Spülflüssigkeits-Vorratstank vorgesehen, dessen Spülflüssigkeit in das Kapillarelektrophorese-Element eingespeist werden kann. Nach einer Elektrophorese muss die Elektrophorese-Kapillare gespült werden. Dies kann unter Umständen mit der Trägerflüssigkeit erfolgen, die auch für die Dialyse verwendet wird. In der Regel ist es jedoch vorteilhaft, eine separate Spülflüssigkeit zum Spülen der Elektrophorese-Kapillare vorzuhalten.

Vorzugsweise ist zwischen der Dialysemembran und dem Kapillarelektrophorese-Element eine Entgasungseinheit zur Entgasung des Wasserdialysats vorgesehen. Die Elektrophorese-Kapillare beziehungsweise die Kapillarelektrophorese ist sehr empfindlich in Bezug auf Gasblasen in dem Wasserdialysat. Daher müssen Gasblasen in dem in die Elektrophorese-Kapillare eingeleiteten Wasserdialysat mit großer Zuverlässigkeit ausgeschlossen werden. Dies erfolgt durch die Entgasungseinheit, die möglichst kurz vor der Eingangs-Öffnung der Elektrophorese-Kapillare angeordnet ist. Die Entgasungseinheit wird beispielsweise durch eine gasdurchlässige Membran gebildet, durch die unter Anlegung von Unterdruck dem Wasserdialysat darin gelöstes Gas entzogen wird. Durch das Vorsehen der Entgasungseinheit wird eine hohe Betriebssicherheit realisiert.

Gemäß einer bevorzugten Ausgestaltung ist der Analyt-Detektor ein Leitfähigkeitsdetektor, und besonders bevorzugt ein kapazitiv gekoppelter kontaktloser Leitfähigkeitsdetektor. Ein Vorteil der berührungslosen Leitfähigkeitsmessung liegt vor allem in dem Schutz der Elektroden vor der Probe. Mit einem derartigen Leitfähigkeitsdetektor können an der Elektrophorese-Kapillare oder unmittelbar hinter ihrem Ausgang die spezifische Wanderungszeit vom Kapillaren-Probenaufgabeort bis zum Detektor und die Menge des betreffenden Analyts kontaktlos festgestellt werden. Das Wasserdialysat kann durch den berührungslosen Analyt-Detektor nicht verändert werden.

Vorzugsweise ist eine Pumpe zum Pumpen der Flüssigkeiten aus dem beziehungsweise den Vorratstanks vorgesehen. Bevorzugt sind die Pumpen hinter dem Vorratstank beziehungsweise den Vorratstanks und vor dem Kapillarelektrophorese-Element angeordnet, so dass sie als Druckpumpen arbeiten.

Vorzugsweise ist das Prozessanalysegerät als eine Baueinheit mit einem Gehäuse ausgebildet, in oder an dem alle vorgenannten Bestandteile bzw. Bauteile angeordnet sind. Das Prozessanalysegerät erfordert vor einer Messung keinen nennenswerten Aufbauaufwand, ist prinzipiell mobil, und im Prinzip nach dem Einschalten betriebsbereit.

Das Prozessanalysegerät ist als Tauchsonde ausgebildet. Alle Bauteile des Prozessanalysegerätes befinden sich also innerhalb der Tauchsonde in einem flüssigkeitsdichten Gehäuse. Hierdurch lassen sich sehr kurze Leitungen, insbesondere eine sehr kurze Probenleitung, realisieren. Durch den Wegfall eines bzw. mehrerer Reagenztanks und gegebenenfalls eines Abfalltanks kann die Tauchsonde relativ klein ausfallen.

Vorzugsweise sind einige oder alle Tanks in einer austauschbaren Kartusche angeordnet. Alle Tanks können auf diese Weise mit einem einzigen Handgriff ausgetauscht werden, wenn die Tankfüllungen erschöpft bzw., im Falle des Abfalltanks, voll sind.

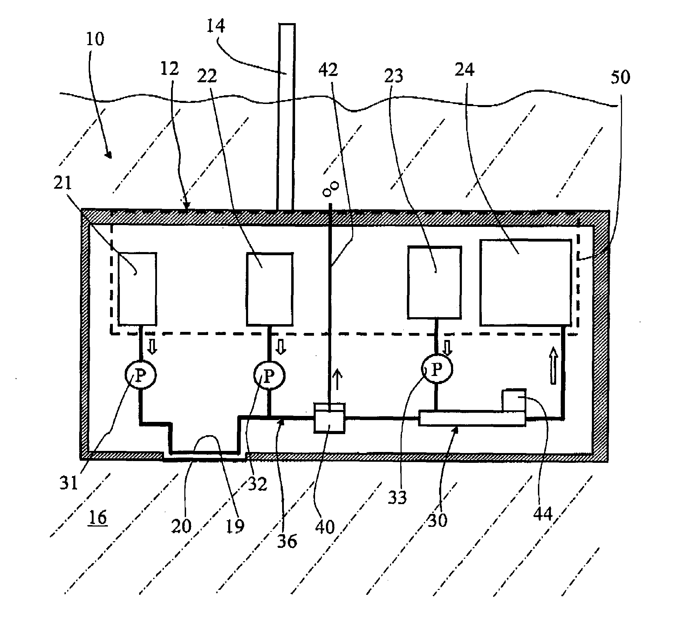

Im Folgenden wird unter Bezugnahme auf die Zeichnung ein Ausführungsbeispiel der Erfindung näher erläutert.

Die Figur zeigt ein als Tauchsonde ausgebildetes Wasser-Prozessanalysegerät, das vorliegend als Abwasser-Prozessanalysegerät ausgebildet ist.

Das in der Figur dargestellte Wasser-Prozessanalysegerät 10 ist eine Tauchsonde, die von einer Haltestange 14 gehalten in Wasser 16 dauerhaft eingetaucht ist. Das Prozessanalysegerät 10 dient vorliegend dazu, ein Wasser-Analyt, beispielsweise Ammonium, Phosphat und/oder Nitrat, In dem Wasser 16 qualitativ und quantitativ zu bestimmen, um eine Regelgröße in einem Wasser-Regelungsprozess zur Verfügung zu stellen und/oder um Messwerte zur Qualitätsüberwachung zu erfassen.

Das Prozessanalysegerät 10 weist ein flüssigkeitsdichtes Gehäuse 12 auf, indem eine Reihe von Bauteilen angeordnet ist. In dem Gehäuse 12 sind vier Tanks 21 -24 angeordnet, nämlich ein Trägerflüssigkeits-Vorratstank 21 ein pH-Pufferlösungs-Vorratstank 22, ein Spülflüssigkeits-Vorratstank 23 und ein Abfalltank 24. Hinter den drei Vorratstanks 21 - 23 ist jeweils eine Flüssigkeitspumpe 31,32,33 angeordnet, die die Flüssigkeit aus dem betreffenden Vorratstank 21-23 zu einer Probenleitung 36 pumpen. Die Pumpen können beispielsweise Peristaltik-Pumpen sein, können jedoch auch anderen Typs sein, insbesondere typische Mikrofluidik-Pumpen sein.

Die Trägerflüssigkeits-Pumpe 31, die eine Probentransport-Einrichtung bildet, pumpt die Trägerflüssigkeit aus dem Vorratstank 21 zu einer Dialysatseite 19 einer Dialysemembran 20. Im Bereich der Dialysemembran 20 diffundieren Analyt-Ionen von der Wasserseite durch die Dialysemembran 20 zur Dialysatseite 19 in die Trägerflüssigkeit hinein, die hierdurch zu einem Wasserdialysat wird. Das Wasserdialysat wird durch die Trägerflüssigkeit-Pumpe 31 weiter in Richtung Kapillarelektrophorese-Element 30 gepumpt. Hinter der Dialysemembran 20 wird eine pH-Pufferlösung aus dem pH-Pufferlösungs-Vorratstank 22 durch die Pumpe 32 in die Probenleitung 36 eingeleitet. Hierdurch wird der pH-Wert des Wasserdialysats stets auf den gleichen Wert eingestellt.

Anschließend fließt das Wasserdialysat durch eine Entgasungseinheit 40, in der in dem Wasserdialysat gelöste Gase dem Wasserdialysat entzogen werden. Hierzu weist die Entgasungseinheit 40 eine gasdurchlässige Membran auf, hinter der durch eine Gaspumpe 41 Vakuum erzeugt wird, durch das das gelöste Gas dem Wasserdialysat entzogen wird. Dieses entzogene Gas wird durch die Gaspumpe 41 über eine Gasleitung 42 aus dem Gehäuse 12 heraus in das Wasser 16 eingeleitet.

Das Wasserdialysat wird anschließend dem Kapillarelektrophorese-Element 30 zugeführt, das im Wesentlichen von einer Elektrophorese-Kapillare gebildet wird. Die Elektrophorese-Kapillare ist mit einem Trennpuffer gefüllt, der die für die Elektrophorese notwendigen Trennbedingungen herstellt, insbesondere bezüglich des pH-Wertes und der Leitfähigkeit der Lösung. In die Elektrophorese-Kapillare wird mittels einer Pumpe oder durch elektrokinetische Injektion ein Probenaliquot injiziert. Üblicherweise erfolgt die Probeninjektion durch elektrokinetische Injektion oder durch eine Pumpe, in der ein winziges Probenaliquot von einigen Nanolitern in die Kapillarelektrophorese-Strecke zwischen zwei Hochspannungselektroden injiziert wird. Dies kann über ein Ventil oder über zwei sich kreuzende Kapillaren erfolgen.

Für die Dauer der Elektrophorese, während der über die Länge der Kapillare eine elektrische Spannung angelegt wird, wird keine Flüssigkeit in die Elektrophorese-Kapillare gepumpt. Durch die angelegte Spannung werden die Ionen elektrophoretisch getrennt.

Am Ende des Kapillarelektrophorese-Elements 30 ist ein Analyt-Detektor 44 angeordnet, der der qualitativen und quantitativen Detektion der elektrophoretisch getrennten Ionen dient. Der Analyt-Detektor 44 ist bevorzugt als kapazitiv gekoppelter kontaktloser Leitfähigkeitsdetektor ausgebildet.

Die zu analysierenden Ionen werden im elektrischen Feld aufgrund Ihrer spezifischen Wanderungsgeschwindigkeit aufgetrennt und wandern während der Elektrophorese am Detektor vorbei. Dort können sie aufgrund Ihrer spezifischen Wanderungszeit qualitativ und aufgrund ihrer aus der Änderung der Ionenkonzentration resultierenden Leitfähigkeitsänderung quantitativ analysiert werden. Auf diese Weise können verschiedene Ionen des Wassers 16 in einem einzigen Messzyklus quantitativ bestimmt werden, ohne dass hierfür auch nur ein einziges Reagenz erforderlich wäre.

Für den folgenden Elektrophorese-Messzyklus wird der Kapillaren-Trennkanal mit neuem Trennpuffer gespült, um ihn auf diese Weise zu säubern und die überschüssige Lösung in den Abfalltank 24 zu pumpen. Daraufhin ist das Kapillarelektrophorese-Element 30 für einen neuen Messzyklus bereit. Die Spülung ist nicht unbedingt nach jedem Messzyklus notwendig. Idealerweise können 3-10 Messzyklen mit dem gleichen Trennpuffer durchgeführt werden.

## Patentansprüche

1. Wasser-Prozessanalysegerät (10) zur kontinuierlichen Bestimmung eines Wasser-Analyts in Wasser (16), mit
einer Dialysemembran (20) mit einer Wasserseite und einer Dialysatseite (19), wobei auf der Dialysatseite (19) ein Wasserdialysat generiert wird,
einem Kapillarelektrophorese-Element (30) zur Trennung des Analyts in dem Wasserdialysat,
einer Probentransport-Einrichtung (31) zum automatischen Transport des Wasserdialysats durch eine Probenleitung (36) von der Dialysemembran (20) zu dem Kapillarelektrophorese-Element (30), und
einem dem Kapillarelektrophorese-Element (30) zugeordneten Analyt-Detektor (44), der das in dem Kapillarelektrophorese-Element (30) getrennte Analyt detektiert, wobei
zwischen der Dialysemembran (20) und dem Kapillarelektrophorese-Element (30) eine Entgasungseinheit (40) zur Entgasung des Wasserdialysats vorgesehen ist,
ein Spülflüssigkeits-Vorratstank (23) vorgesehen ist, dessen Spülflüssigkeit in das Kapillarelektrophorese-Element (30) eingespeist werden kann, wobei die Einspeisung der Spülflüssigkeit in Strömungsrichtung hinter der Entgasungseinheit 40 erfolgt, und
das Prozessanalysegerät (10) eine Baueinheit mit einem Gehäuse (12) ist, in oder an dem alle genannten Bestandteile angeordnet sind, wobei das Prozessanalysegerät (10) als Tauchsonde ausgebildet ist.

2. Wasser-Prozessanalysegerät (10) nach Anspruch 1, wobei ein Transportflüssigkeits-Vorratstank (21) zur Versorgung der ionenselektiven Dialysemembran (20) mit einer Transportflüssigkeit vorgesehen ist.

3. Wasser-Prozessanalysegerät (10) nach Anspruch 1 oder 2, wobei ein pH-Pufferlösungs-Vorratstank (22) vorgesehen ist, dessen Pufferlösung zwischen der Dialysemembran (20) und dem Kapillarelektrophorese-Element (30) in die Probenleitung (36) eingeleitet wird.

4. Wasser-Prozessanalysegerät (10) nach einem der vorangehenden Ansprüche, wobei der Analyt-Detektor (44) ein Leitfähigkeitsdetektor ist.

5. Wasser-Prozessanalysegerät (10) nach einem der vorangehenden Ansprüche, wobei der Analyt-Detektor (44) ein kapazitiv gekoppelter kontaktloser Leitfähigkeitsdetektor ist.

6. Wasser-Prozessanalysegerät (10) nach einem der vorangehenden Ansprüche, wobei mindestens eine Pumpe (31, 32, 33) zum Pumpen der Flüssigkeiten aus den Vorratstanks (21, 22, 23) vorgesehen ist.

7. Wasser-Prozessanalysegerät (10) nach einem der vorangegangenen Ansprüche, wobei ein oder mehrere Tanks (21,22,23,24) in einer austauschbaren Kartusche (50) vorgesehen sind.

## Claims

1. Water process analysis device (10) for continuously detecting a water analyte in water (16), comprising
a dialysis membrane (20) having a water side and a dialysate side (19), water dialysate being generated on the dialysate side (19),
a capillary electrophoresis element (30) for the separation of the analyte in the water dialysate,
a sample transport means (31) for the automatic transport of said water dialysate through a sample line (36) from the dialysis membrane (20) to the capillary electrophoreses element (30), and
an analyte detector (44) assigned to the capillary electrophoresis element (30), the detector detecting the analyte separated in the capillary electrophoresis element (30), wherein
a degassing unit (40) for degassing the water dialysate is provided between the dialysis membrane (20) and the capillary electrophoresis element (30),
a rinsing liquid reservoir (23) is provided, whose rinsing liquid can be supplied into the capillary electrophoresis element (30), the rinsing liquid being supplied downstream of the degassing unit (40), seen in the direction of flow, and
said process analysis device (10) is a structural unit with a housing (12) in or at which all components mentioned are arranged, said process analysis device (10) being configured as a, immersion probe.

2. Water process analysis device (10) of claim 1, wherein a transport fluid reservoir (21) is provided for supplying the ion-selective dialysis membrane (20) with a transport liquid.

3. Water process analysis device (10) of claim 1 or 2, wherein a pH buffer solution reservoir (22) is provided, whose buffer solution is introduced into the sample line (36) at a position between the dialysis membrane (20) and the capillary electrophoresis element (30).

4. Water process analysis device (10) of one of the preceding claims, wherein the analyte detector (44) is a conductivity detector.

5. Water process analysis device (10) of one of the preceding claims, wherein the analyte detector (44) is a capacitively coupled contactless conductivity detector.

6. Water process analysis device (10) of one of the preceding claims, wherein at least one pump (31, 32, 33) is provided for pumping the liquids from the reservoirs (21, 22, 23).

7. Water process analysis device (10) of one of the preceding claims, wherein one or a plurality of reservoirs (21, 22, 23, 24) are provided in a replaceable cartridge (50).

## Revendications

1. Appareil d'analyse de processus à l'eau (10) pour la détermination continue d'un analyte d'eau (16) dan l'eau, comprenant
une membrane de dialyse (20) avec un côté eau et un côté dialysate (19), un dialysate d'eau étant généré sur le côté dialysate (19),
un élément d'électrophorèse capillaire (30) pour séparer l'analyte dans le dialysate d'eau,
un moyen de transport d'échantillon (31) pour le transport automatique dudit analyte d'eau à travers une conduite d'échantillon (36) à partir de la membrane de dialyse (20) jusqu'à l'élément d'électrophorèse capillaire (30), et
un détecteur d'analyte (44) assigné à l'élément d'électrophorèse capillaire (30), ledit détecteur détectant l'analyte séparé dans ledit élément d'électrophorèse capillaire (30),
une unité de dégazage (40) étant prévue entre ladite membrane de dialyse (20) et ledit élément d'électrophorèse capillaire (30), pour dégazer le dialysate d'eau,
un réservoir de liquide de rinçage (23) étant prévu, dont ladite liquide de rinçage peut être introduite dans ledit élément d'électrophorèse capillaire (30), la liquide de rinçage étant introduite en aval de ladite unité de dégazage (40), vu dans la direction d'écoulement, et
ledit appareil d'analyse de processus (10) étant une unité structurelle avec un boitier (12) dans ou sur lequel tous les composants mentionnés sont disposés, ledit appareil d'analyse de processus (10) étant réalisé comme sonde immergée.

2. Appareil d'analyse de processus à l'eau (10) selon la revendication 1, dans lequel un réservoir de liquide de transport (21) est prévu pour alimenter ladite membrane de dialyse (20) sélective aux ions en une liquide de transport.

3. Appareil d'analyse de processus à l'eau (10) selon les revendications 1 ou 2, dans lequel un réservoir d'une solution-tampon pH (22) est prévu, dont ladite solution-tampon est introduite dans ladite conduite d'échantillon (36) à une position entre ladite membrane de dialyse (20) et ledit élément d'électrophorèse capillaire (30).

4. Appareil d'analyse de processus à l'eau (10) selon l'une quelconque des revendications précédentes, dans lequel ledit détecteur d'analyte (44) est un détecteur de conductivité.

5. Appareil d'analyse de processus à l'eau (10) selon l'une quelconque des revendications précédentes, dans lequel ledit détecteur d'analyte (44) est un détecteur de conductivité sans contact, couplé capacitivement.

6. Appareil d'analyse de processus à l'eau (10) selon l'une quelconque des revendications précédentes, dans lequel au moins une pompe (31, 32, 33) est prévue pour pomper les liquides desdits réservoirs (21, 22, 23).

7. Appareil d'analyse de processus à l'eau (10) selon l'une quelconque des revendications précédentes, dans lequel un ou plusieurs réservoirs (21, 22, 23, 24) sont prévus dans une cartouche (50) échangeable.
